# FASCICULE DE BREVET EUROPEEN

(11) **EP 3 386 490 B1**
(45) Date de publication et mention de la délivrance du brevet: **14.04.2021**
(21) Numéro de dépôt: 16819596.4
(22) Date de dépôt: 02.12.2016
(51) Int. Cl.: A61K 9/70, A61K 9/16, A61K 9/00, A61K 31/702, A61P 17/02

(54) **FILM COMPRENANT UN OLIGOSACCHARIDE POLYSULFATÉ ET UN POLYCATION ET SON PROCÉDÉ DE FABRICATION**
FILM MIT EINEM POLYSULFATIERTEM OLIGOSACCHARID UND EINEM POLYKATION UND VERFAHREN ZUR HERSTELLUNG DAVON
FILM INCLUDING A POLYSULFATED OLIGOSACCHARIDE AND A POLYCATION AND METHOD FOR MANUFACTURING SAME

(30) Priorité: 07.12.2015 FR 1561928
(43) Date de publication de la demande: 17.10.2018
(73) Titulaire: Urgo Recherche Innovation et Développement, 21300 Chenove (FR); Centre National de la Recherche Scientifique (CNRS), 75016 Paris (FR)
(72) Inventeur: BOULMEDAIS, Fouzia, 67000 Strasbourg (FR); SCHAAF, Pierre, 67120 Molsheim (FR); PERNOT, Jean-Marc, 21000 Dijon (FR); LAURENSOU, Christelle, 21000 Dijon (FR)
(74) Mandataire: Plasseraud IP
(86) Numéro de dépôt international: PCT/FR2016/053191
(87) Numéro de publication internationale: WO 2017/098118

(56) Documents cités:
- EP-A1- 2 082 869
- EP-A1- 2 695 621
- WO-A1-98/22114
- WO-A1-2013/007961
- FR-A1- 2 967 580
- Richert Ludovic: "FILMS MULTICOUCHES A BASE DE POLYELECTROLYTES BIODEGRADABLES : CARACTERISATION PHYSICO-CHIMIQUE ET INTERACTION AVEC DES CELLULES", , 26 novembre 2009 (2009-11-26), XP055290828, Extrait de l'Internet: URL:http://docnum.univ-lorraine.fr/public/ SCD_T_2004_0251_RICHERT.pdf [extrait le 2016-07-25]
- DECHER G ET AL: "Buildup of ultrathin multilayer films by a self-assembly process: III. Consecutively alternating adsorption of anionic and cationic polyelectrolytes on charged surfaces", THIN SOLID FILMS, ELSEVIER, AMSTERDAM, NL, vol. 210-211, 30 avril 1992 (1992-04-30), pages 831-835, XP024731651, ISSN: 0040-6090, DOI: 10.1016/0040-6090(92)90417-A [extrait le 1992-04-30]

## Description

La présente invention a pour objet un nanofilm comprenant au moins un polycation et un actif. Cet actif est choisi parmi les oligosaccharides polysulfatés synthétiques ayant 1 à 4 unités oses, leurs sels, ou leurs complexes. La présente invention porte également sur les nanofilms susceptibles d'être obtenus par pulvérisation, simultanée ou en alternance, d'au moins une solution de polycation et d'au moins une solution d'oligosaccharide polysulfaté ainsi que l'utilisation de ces nanofilms sur la peau, les affections cutanées, la plaie ou les muqueuses. La présente invention porte également sur un nanofilm pour son utilisation dans une méthode de libération d'oligosaccharide polysulfaté au contact des plaies exsudatives.

La cicatrisation d'une plaie est un phénomène biologique naturel, les tissus humains et animaux étant capables de réparer des lésions localisées par des processus de réparation et de régénération qui leur sont propres.

La rapidité et la qualité de la cicatrisation d'une plaie dépendent de l'état général de l'organisme atteint, de l'étiologie de la plaie, de l'état et de la localisation de la plaie, et de la survenue ou non d'une infection, ainsi que des facteurs génétiques prédisposant ou non à des troubles de la cicatrisation.

La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de formation de la cicatrice.

La première phase, la phase inflammatoire, débute dès la rupture des vaisseaux sanguins qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple cytokine, facteurs de croissance) permettant le recrutement des cellules de la cicatrisation comme les cellules inflammatoires (les polynucléaires neutrophiles et les macrophages), les fibroblastes et les cellules endothéliales.

La seconde phase correspond au développement du tissu de granulation. On observe d'abord une colonisation de la blessure par prolifération des fibroblastes. Puis, la migration des cellules endothéliales à partir des vaisseaux sains va permettre la néovascularisation, ou angiogénèse, du tissu lésé. Dans le tissu de granulation, les fibroblastes sont activés et vont se différencier en myofibroblastes présentant des propriétés contractiles importantes, générées par les microfilaments d'actine, permettant la contraction de la plaie. Ces microfilaments sont exprimés par une protéine : l'actine α-musculaire lisse. Ces myofibroblastes jouent donc un rôle majeur dans la formation et la maturation du tissu de granulation qui va conduire à la cicatrisation de la lésion. Il y a ensuite migration des kératinocytes et reconstruction de l'épiderme.

La troisième phase du processus de réparation, la formation de la cicatrice ou maturation, s'accompagne d'un remodelage du tissu de granulation. Une partie de la matrice extracellulaire est digérée par des protéases (essentiellement des métallo-protéases matricielles (MMP) et des élastases), et on observe une réorganisation progressive de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis les cellules excédentaires meurent par apoptose. Parallèlement au remodelage de la matrice extracellulaire et à l'apoptose des cellules excédentaires, l'état inflammatoire diminue progressivement. Cette phase est la plus longue : au bout d'un an environ, la cicatrice se remodèle, elle n'est plus rouge, ni rigide, et ne provoque plus douleur et elle s'aplanie.

Les oligosaccharides polysulfatés sont connus et utilisés pour traiter diverses pathologies de la peau, et notamment pour le traitement des plaies. A titre d'exemple, on peut citer les produits de la gamme « Cicalfate », commercialisés par les Laboratoires Pierre Fabre pour favoriser la réparation de l'épiderme qui contiennent un oligosaccharide polysulfaté, le sucralfate. Ces produits se présentent sous forme de crème.

On peut également citer les produits « UrgoStart » commercialisés par les Laboratoires Urgo pour le traitement des plaies qui contiennent un oligosaccharide polysulfaté, le sucrose octasulfate de potassium. Ces produits se présentent sous la forme de pansements. Le sucrose octasulfate de potassium est incorporé à la matrice lipido-colloïde.

La présente invention porte sur un nouveau mode d'incorporation des oligosaccharides polysulfatés sous forme de nanofilms permettant leur libération lorsqu'ils sont en contact avec la peau, la plaie ou les muqueuses.

En particulier, l'invention a pour objet un nanofilm caractérisé en ce qu'il comprend au moins un polycation et un oligosaccharide polysulfaté ayant une à quatre unités ose.

Les nanofilms de l'invention sont susceptibles d'être obtenus par pulvérisation d'au moins une solution de polycation et d'au moins une solution d'oligosaccharide polysulfaté.

On entend par nanofilm au sens de la présente invention des films présentant une épaisseur comprise entre 1 nm et 10 µm, de préférence inférieure à 10 µm. Selon un mode de réalisation préféré, les nanofilms de l'invention présentent une épaisseur de 10 nm à 8 µm, plus préférentiellement de 50 nm à 5 µm, plus préférentiellement encore de 100 nm à 1 µm.

Le nanofilm selon l'invention est caractérisé en ce qu'il comprend au moins un polycation et au moins un oligosaccharide polysulfaté.

Les nanofilms selon la présente invention sont susceptibles d'être obtenus par pulvérisation, simultanée ou en alternance, d'au moins une solution de polycation et d'au moins une solution d'oligosaccharide polysulfaté.

Les nanofilms de l'invention sont particulièrement intéressants lorsqu'ils sont appliqués sur une plaie. En effet, au contact des exsudats, le nanofilm se solubilise rapidement permettant la libération des oligosaccharides polysulfatés. Ceci peut permettre une action immédiate au contact des exsudats et donc d'atteindre la dose efficace plus rapidement. En outre, les nanofilms de l'invention contiennent une quantité d'oligosaccharide polysulfaté optimale pour le traitement des plaies tout en limitant les quantités des matières premières utilisées dans leur procédé de fabrication.

La présente invention a également pour objet un nanofilm pour son utilisation dans une méthode de libération d'oligosaccharide polysulfaté au contact des plaies exsudatives, ladite méthode comprenant en particulier une première étape de préparation d'un nanofilm selon l'invention, et une seconde étape de mise en contact du nanofilm avec une plaie exsudative.

Ainsi, la présente invention s'intéresse plus particulièrement à l'utilisation des nanofilms contenant le polycation et les oligosaccharides polysulfatés pour le traitement des plaies exsudatives. En effet, en présence des exsudats, les nanofilms se dissolvent et libèrent l'actif qu'ils contiennent.

### Oligosaccharides polysulfatés

Les oligosaccharides utilisés dans le cadre de la présente invention sont des oligomères synthétiques formés de 1 à 4 unités d'oses, et de préférence de 1 ou 2 unités d'oses, généralement liées entre elles par liaison glycosidique alpha ou bêta. En d'autres termes, il s'agit de mono, di, tri ou tétrasaccharides, et de préférence de mono ou disaccharides.

Il n'y a pas de limitation particulière concernant la nature des unités oses de ces polysaccharides. De préférence, il s'agira de pentoses ou d'hexoses. A titre d'exemple de monosaccharide, on peut citer le glucose, le galactose ou le mannose. A titre d'exemple de disaccharide, on peut citer le maltose, le lactose, le sucrose ou le tréhalose. A titre d'exemple de trisaccharide, on peut citer le mélézitose. A titre d'exemple de tétrasaccharide, on peut citer le stachyose.

De préférence, l'oligosaccharide est un disaccharide, et de préférence encore le sucrose. On entend par "oligosaccharide polysulfaté" au sens de la présente demande un oligosaccharide dont au moins deux, et de préférence tous les groupes hydroxyles de chaque ose ont été substitués par un groupe sulfate.

De préférence, l'oligosaccharide polysulfaté utilisé dans le cadre de la présente demande est le sucrose octasulfate.

Les oligosaccharides polysulfatés utilisés dans le cadre de la présente invention peuvent se présenter sous forme de sels ou complexes.

A titre d'exemple de sels, on peut citer les sels de métal alcalin tels que les sels de sodium, de calcium ou de potassium; les sels d'argent ; ou encore les sels d'acide aminé.

A titre d'exemple de complexes, on peut citer les complexes d'hydroxyaluminium.

Dans le cadre de la présente invention, des composés particulièrement préférés sont les suivants :
- le sel de potassium du sucrose octasulfaté (ou sucrose octasulfate de potassium) ;
- le sel d'argent du sucrose octasulfaté ; et
- le complexe hydroxyaluminium du sucrose octasulfate, appelé communément sucralfate.

Le sucrose octasulfate de potassium (ou KSOS) est particulièrement préféré. Il se présente sous la forme d'une molécule multichargée qui présente des propriétés inhibitrices des Métallo Protéases Matricielles. Il se présente sous forme de petite chaîne polymérique chargée négativement (polyanion).

Dans le cadre de la présente invention, les oligosaccharides polysulfatés sont utilisés dans des quantités allant de 10 % à 90%, de préférence de 30 à 70 %, en poids par rapport au poids du nanofilm.

En particulier, dans le cadre de la présente invention, les oligosaccharides polysulfatés utilisés sont de préférence les sels de potassium.

Les oligosaccharides polysulfatés utilisés dans le cadre de la présente invention peuvent se présenter sous forme de poudre micronisée ou sous forme solubilisée.

Le sel de potassium du sucrose octasulfate est connu depuis 25 ans pour le traitement des plaies lors de la phase de bourgeonnement grâce à son action sur les fibroblastes. Cette action est par exemple décrite dans les demandes de brevet EP 230 023, WO 89/05645 ou WO 98/22114. Comme il est précisé dans ces documents, ce composé est utilisé après avoir réalisé une détersion assistée de la plaie et donc après avoir éliminé les tissus nécrotiques et/ou fibrineux (voir EP 230 023, WO 89/05645, WO 98/22114). On l'utilise donc sur une plaie propre et détergée.

Dans la demande de brevet internationale WO 89/05645, il est précisé que les polysaccharides polysulfatés tels que le sucralfate sont des inhibiteurs de protéases, notamment les hyaluronidases, qui sont des enzymes capables de dégrader l'acide hyaluronique et les glycosaminoglycanes.

Un des développements actuels des biomatériaux s'oriente vers la réalisation de matériaux bioactifs intelligents. Différentes techniques de traitement de surface ont été développées dans ce but.

### Polycation

Une voie de fonctionnalisation, basée sur le dépôt couche par couche de polyélectrolytes (alternativement un polycation et un polyanion), a été proposée au début des années 1990.

L'épaisseur des nanofilms obtenus peut être contrôlée en faisant varier plusieurs paramètres (nombre de couches, masses moléculaires, concentrations et nature des polyélectrolytes, pH, force ionique). Leur intérêt se révèle dans le domaine des biomatériaux par la possibilité de leur conférer une fonctionnalité biologique par insertion de protéines, de peptides ou de médicaments. Les films multicouches sont obtenus principalement par deux techniques : par imprégnations alternées dans les solutions de polyélectrolytes ou par pulvérisations alternées ou simultanées de ces solutions. La cohésion de ces films est basée principalement sur les interactions électrostatiques entre les charges positives et négatives des chaînes de polycation et de polyanion mais également entre polyélectrolytes et petites molécules multichargées.

Dans le cadre de la présente invention, le nanofilm comprend au moins un polycation, c'est-à-dire un polymère ionique chargé positivement.

Les nanofilms de l'invention sont obtenus en combinant l'oligosaccharide polysulfaté à des polycations à grandes chaînes.

De préférence, les polycations mis en œuvre peuvent être choisis parmi la poly(L-lysine), le chitosan, le collagène, le poly(éthylène imine) branché, la poly(L-arginine), le poly(allylamine) (PAH), et leurs mélanges ; en particulier la poly(L-lysine) et le poly(éthylène imine) branché.

La cohésion de ces nanofilms est basée sur des interactions électrostatiques entre les charges positives des chaînes de polycation et les charges négatives du polyanion oligosaccharide polysulfaté.

Ces polycations sont de préférence présents dans les nanofilms de l'invention dans des quantités allant de 10 à 90 %, de préférence de 30 à 70% en poids par rapport au poids du nanofilm.

### Milieu pharmaceutiquement acceptable

Le nanofilm de l'invention est en particulier susceptible d'être obtenu par pulvérisation (ou sprayage), simultanée ou en alternance, d'au moins une solution de polycation et d'au moins une solution d'oligosaccharide polysulfaté. En particulier, le nanofilm peut être obtenu par une succession de pulvérisations alternant, à plusieurs reprises, la pulvérisation d'une solution de polycation et la pulvérisation d'une solution d'oligosaccharide polysulfaté.

Le polycation et l'oligosaccharide polysulfaté sont tels que décrits précédemment.

Le nanofilm de l'invention est en particulier susceptible d'être obtenu par 1 à 500 étapes de pulvérisation de la solution de polycation et de la solution d'oligosaccharide polysulfaté, les deux solutions étant pulvérisées de manière simultanée ou en alternance, de préférence de manière simultanée.

En effet, la pulvérisation simultanée des solutions de polycation et d'oligosaccharide polysulfaté peut notamment permettre d'atteindre l'épaisseur souhaitée avec un nombre réduit d'étape par rapport à la pulvérisation alternée. De plus, l'épaisseur du nanofilm obtenu par pulvérisation simultanée est plus facile à contrôler de manière reproductible par rapport à la pulvérisation alternée. Ainsi, la pulvérisation alternée permet d'optimiser la quantité d'oligosaccharide polysulfaté introduite dans le nanofilm.

De préférence, le nanofilm est obtenu par 5 à 200 étapes de pulvérisation, et plus préférentiellement par 20 à 100 étapes de pulvérisation de la solution de polycation et de la solution d'oligosaccharide polysulfaté.

Les solutions liquides comprenant le polycation ou l'oligosaccharide polysulfaté comprennent notamment un milieu pharmaceutiquement acceptable pouvant contenir de l'eau.

Par milieu pharmaceutiquement acceptable, on entend, au sens de la présente demande, un milieu compatible avec les polycations et/ou les oligosaccharides polysulfatés.

L'eau peut notamment être présente dans les solutions liquides comprenant le polycation ou l'oligosaccharide polysulfaté en une teneur supérieure à 70%, de préférence supérieure à 95%.

En particulier, les solutions liquides comprenant le polycation ou l'oligosaccharide polysulfaté peuvent comprendre un mélange d'eau (i.e. à titre de milieu pharmaceutiquement acceptable) et de chlorure de sodium (NaCl), « sel de fond », de préférence à une concentration de 150 mM.

Chaque étape de pulvérisation peut être conduite pendant une durée allant d'1s à 1min, de préférence de 5 à 10s.

Le débit de pulvérisation peut, par exemple, être compris entre 0.1 et 1 mL/s, de préférence entre 0.2 et 0.5 mL/s.

Chaque étape de pulvérisation est de préférence suivie d'une étape de rinçage et/ou d'une étape de séchage.

Chaque étape de rinçage et chaque étape de séchage est de préférence conduite pendant une durée allant d'1s à 1min, de préférence de 5 à 10s.

Le nanofilm de l'invention peut également être obtenu par un procédé d'imprégnation alternée (rolling/dipping) dans au moins une solution de polycation et au moins une solution d'oligosaccharide polysulfaté.

Le polycation et l'oligosaccharide polysulfaté sont tels que décrits précédemment.

Le nanofilm de l'invention est en particulier susceptible d'être obtenu par 1 à 500 étapes d'imprégnation alternée dans la solution de polycation et la solution d'oligosaccharide polysulfaté. De préférence, le nanofilm est obtenu par 5 à 200 étapes d'imprégnation alternée, et plus préférentiellement par 20 à 100 étapes d'imprégnation alternée dans la solution de polycation et la solution d'oligosaccharide polysulfaté.

Les solutions liquides comprenant le polycation ou l'oligosaccharide polysulfaté comprennent notamment un milieu pharmaceutiquement acceptable pouvant contenir de l'eau.

L'eau peut notamment être présente dans les solutions liquides comprenant le polycation ou l'oligosaccharide polysulfaté en une teneur supérieure à 70%, de préférence supérieure à 95%.

En particulier, les solutions liquides comprenant le polycation ou l'oligosaccharide polysulfaté peuvent comprendre un mélange d'eau (i.e. à titre de milieu pharmaceutiquement acceptable) et de chlorure de sodium (NaCl), « sel de fond », de préférence à une concentration de 150 mM.

Chaque étape d'imprégnation peut être conduite pendant une durée allant de 1 min à 15 min, de préférence de 5 min à 10 min.

Chaque étape d'imprégnation est de préférence suivie d'une étape de rinçage et/ou d'une étape de séchage.

Chaque étape de rinçage est de préférence conduite pendant une durée allant de 1 min à 15 min, de préférence de 5 min à 10 min.

Chaque étape de séchage est de préférence conduite pendant une durée allant d'1s à 1min, de préférence de 5 à 10s.

L'invention a également pour objet la combinaison de compositions liquides caractérisée en ce qu'elle comprend au moins une solution de polycation et au moins une solution d'oligosaccharide polysulfaté précédemment décrites.

L'invention est illustrée plus en détails dans les exemples non limitatifs suivants.

### Exemples de nanofilms selon l'invention :

Les polycations qui ont été utilisés sont d'origine synthétique, comme la **poly(L-lysine)** (polypeptide) ou d'origine naturelle, comme le collagène (**COL**, protéine).

Les solutions de sucrose octasulfate de potassium (ou KSOS) et de polycations ont été préparées dans de l'eau ultra-pure (de résistivité 18.2 MΩ.cm, système Milli-Q-plus, Millipore).

La construction des nanofilms a été réalisée sur des substrats en silicium (Wafernet INC, USA), préalablement découpés à l'aide d'une pointe diamant en rectangles d'environ 2×2 cm². Avant utilisation, les substrats ont été traités au plasma cleaner (Harrick Plasma, USA) afin d'éliminer les impuretés et de les rendre hydrophiles. Le gaz utilisé est l'oxygène contenu dans l'air.

Quel que soit le système étudié, une première couche de poly(éthylène imine) branché (BPEI) a été déposée par trempage (5 minutes) du substrat dans une solution aqueuse de BPEI suivi par deux rinçages à l'eau (d'une minute chacun) et un séchage à l'azote. Cette première couche sert de couche d'accroche favorisant la construction des nanofilms.

La préparation des nanofilms polycation/KSOSt par pulvérisation est décrite ci-dessous. Un appareil de pulvérisation comprenant quatre aérographes, reliés à une arrivée d'air comprimé et placés à une distance de 25 cm du substrat a été utilisé. Deux aérographes permettent de pulvériser les deux composés en solution (KSOS et le polycation choisi). Deux autres permettent d'effectuer les étapes de rinçage et de séchage des dépôts.

Afin d'évaluer la quantité de composés pulvérisés, les débits de pulvérisations sont mesurés lors de chaque manipulation et sont de l'ordre de 0,3 mL/s.

Le substrat est mis en rotation pendant la durée des pulvérisations à environ 1000 tours/min afin d'obtenir des dépôts homogènes.

Plusieurs paramètres ont été étudiés :
- le type de pulvérisation (alternée ou simultanée)
- la concentration en sel de fond NaCl (0 et 150 mM)
- le type de polycations (d'origine synthétique ou naturelle)
- le rapport KSOS/polycation pulvérisé

Afin de mesurer l'épaisseur des dépôts successifs sur les substrats de silicium, l'ellipsométrie a été utilisée.

Les tests de stabilité des nanofilms ont été réalisés en mettant un substrat de 2 × 2 cm² au contact de 20 mL de milieu physiologique et de milieu simulant la plaie pendant 45 min. Le milieu physiologique est modélisé par un tampon phosphate à pH 7.4 ± 0.1 (phosphate buffer saline, PBS) obtenu par dilution d'une solution concentré PBS 10× contenant du Ca²⁺ et du Mg²⁺.

Le milieu de la plaie est modélisé par une solution aqueuse 5% massique NaCl et 5% massique de CaCl₂ dihydraté.

### Exemple 1 selon l'invention : nanofilms de Poly(L-lysine)/KSOS

Les nanofilms de PLL/KSOS ont été obtenus après 25 étapes de pulvérisation simultanée. Les durées de pulvérisations sont les suivantes : 10 s pour les composés pulvérisés ; 5 s pour le rinçage, 5 s pour le séchage.

Les deux composés sont utilisés en solution aqueuse avec 150 mM NaCl en sel de fond. L'optimisation de la construction a été obtenue en faisant varier la concentration en KSOS de 0,05 à 12 mg/mL pour une concentration en PLL de 0,2 mg/mL.

L'optimum de construction est obtenu avec 0,2 mg/mL en KSOS.

Après l'élaboration des nanofilms PLL/KSOS, leur stabilité a été testée au contact du milieu physiologique et au contact du milieu de la plaie. Pour ce faire, chaque nanofilm d'une taille de 2 × 2 cm² a été mis au contact de 750 µL de milieu physiologique ou de milieu de la plaie pendant 45 min suivi d'une étape de rinçage à l'eau Milli Q pendant 45 min.

Les nanofilms PLL/KSOS présentent une faible perte d'épaisseur (< 10%) au contact du milieu physiologique, ce qui signifie que les nanofilms sont stables au contact du milieu physiologique. De plus, la spectroscopie infrarouge montre qu'une faible partie du KSOS est libérée lors de ce contact : la bande à 1240 cm⁻¹ du KSOS diminue faiblement.

Par contre au contact du milieu de la plaie, les nanofilms PLL/KSOS sont totalement dissous, libérant ainsi la totalité du KSOS.

### Exemple 2 selon l'invention : étude de la pulvérisation dans l'obtention de nanofilms de Poly(L-lysine)/KSOS

Dans cet exemple, les deux composés (PLL et KSOS) sont utilisés en solution aqueuse avec 150 mM NaCl en sel de fond et à une concentration de 0.2 mg/mL.

Les nanofilms de PLL/KSOS ont été obtenus après 16 étapes de pulvérisation alternée ou simultanée.

Une étape de pulvérisation alternée est effectuée de la manière suivante : la solution de PLL puis la solution de KSOS est pulvérisée pendant 5 s suivi par une étape de rinçage de 5 s et de séchage de 5 s.

Une étape de pulvérisation simultanée est effectuée de la manière suivante : la solution de PLL et de KSOS sont pulvérisée simultanément pendant 5 s suivi par une étape de rinçage de 5 s et de séchage de 5 s.

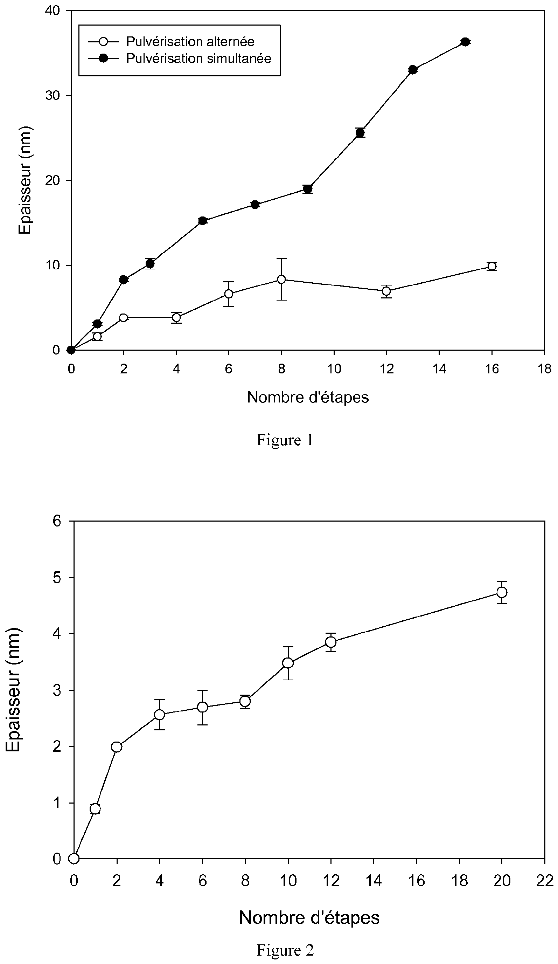

La Figure 1 représente l'évolution de l'épaisseur en nm des nanofilms PLL/KSOS en fonction du nombre d'étapes de pulvérisation alternée et simultanée en présence de 150 mM NaCl. Après 16 étapes de pulvérisation, le nanofilm PLL/KSOS atteint 36 nm par le procédé de pulvérisation simultanée et 10 nm par le procédé de pulvérisation alternée.

Le procédé de pulvérisation simultanée permet d'obtenir des nanofilms PLL/KSOS d'une épaisseur donnée avec moins d'étapes qu'en pulvérisation alternée. En effet, il suffit de 3 étapes en pulvérisation simultanée pour obtenir un nanofilm de 10 nm alors qu'il en faut 16 en pulvérisation alternée. Ainsi, le procédé de pulvérisation simultanée est économiquement avantageux (plus rapide et permet d'utiliser des quantités inférieures de PLL et KSOS).

En outre, les épaisseurs obtenues en pulvérisation simultanée ont une meilleure reproductibilité (barre d'erreur de l'épaisseur inférieure à 10% de l'épaisseur). Ainsi, l'épaisseur du nanofilm est plus facile à contrôler avec la pulvérisation simultanée.

De plus, les épaisseurs obtenues en pulvérisation alternée atteignent une valeur limite de 10 nm sur le nombre d'étapes étudié. Cela veut dire qu'entre 6 et 16 étapes, l'épaisseur n'augmente plus.

En conclusion, la pulvérisation simultanée permet un contrôle plus efficace de la quantité de KSOS introduite dans le nanofilm par rapport à la pulvérisation alternée.

### Exemple 3 selon l'invention : Nanofilms de poly(éthylène imine) branché (BPEI)/KSOS obtenus par imprégnation alternée

Les deux composés (BPEI et KSOS) sont utilisés en solution aqueuse avec 150 mM NaCl en sel de fond et à une concentration de 0.2 mg/mL.

Les nanofilms de BPEI/KSOS ont été obtenus après 20 étapes d'imprégnation alternée. Une étape d'imprégnation est effectuée de la manière suivante : le support est trempé dans une des solutions (BPEI ou KSOS) pendant 5 minutes suivi par une étape de rinçage (trempage dans une solution de 150 mM NaCl) de 5 minutes et de séchage de 5 s.

La Figure 2 représente l'évolution de l'épaisseur en nm des nanofilms BPEI/KSOS en fonction du nombre d'étapes d'imprégnation alternée en présence de 150 mM NaCl. Après 20 étapes d'imprégnation alternée, le nanofilm BPEI/KSOS atteint 4,7 nm. A titre de comparaison, l'épaisseur de nanofilms BPEI/KSOS atteint environ 15 nm par pulvérisation alternée et simultanée. Un des avantages du procédé d'imprégnation est le volume utilisé en solution de composés. En effet pour 20 étapes de dépôts, le volume utilisé pour chaque composé est de 10 mL en imprégnation alternée et 30 mL en pulvérisation alternée et simultanée.

## Revendications

1. Film **caractérisé en ce qu'**il comprend au moins un polycation et un oligosaccharide polysulfaté ayant une à quatre unités ose, ledit film présentant une épaisseur entre 1 nm et 10 µm.

2. Film selon la revendication 1, **caractérisé en ce que** le polycation est présent dans une quantité de 10 à 90 % en poids, de préférence 30 à 70% et l'oligosaccharide polysulfaté dans une quantité de10 à 90 % en poids de préférence 30 à 70%, par rapport au poids du film.

3. Film selon la revendication1 ou 2, **caractérisé en ce que** le polycation est choisi parmi la poly(L-lysine), le chitosan, le collagène, le poly(éthylène imine) branché, la poly(L-arginine), le poly(allylamine), et leurs mélanges ; en particulier la poly(L-lysine) et le poly(éthylène imine) branché.

4. Film selon l'une quelconque des revendications 1 à 3, **caractérisé en ce que** l'oligosaccharide polysulfaté est choisi parmi les sucroses octasulfate, de préférence, le sucrose octasulfate de potassium.

5. Film selon l'une quelconque des revendications 1 à 4, **caractérisé en ce qu'**il présente une épaisseur de 10 nm à 8 µm, plus préférentiellement de 50 nm à 5 µm, plus préférentiellement encore de 100 nm à 1 µm.

6. Film **caractérisé en ce qu'**il est susceptible d'être obtenu par pulvérisation, simultanée ou en alternance, d'au moins une solution de polycation et d'au moins une solution d'oligosaccharide polysulfaté ayant une à quatre unités ose, ledit film présentant une épaisseur entre 1 nm et 10 µm.

7. Film selon la revendication 6, **caractérisé en ce que** les solutions pulvérisées comprennent un milieu pharmaceutiquement acceptable, de préférence l'eau.

8. Film selon la revendication 6 ou 7, **caractérisé en ce que** les pulvérisations sont réalisés de manière simultanée.

9. Film **caractérisé en ce qu'**il est susceptible d'être obtenu par imprégnation alternée dans au moins une solution de polycation et au moins une solution d'oligosaccharide polysulfaté ayant une à quatre unités ose, ledit film présentant une épaisseur entre 1 nm et 10 µm.

10. Film selon la revendication 9, **caractérisé en ce que** les solutions comprennent un milieu pharmaceutiquement acceptable, de préférence l'eau.

11. Film pour son utilisation dans une méthode de libération d'oligosaccharide polysulfaté au contact des plaies exsudatives, ladite méthode comprenant en particulier une première étape de préparation d'un film selon l'une quelconque des revendications 1 à 10, et une seconde étape de mise en contact du film avec une plaie exsudative.

12. Utilisation du film selon l'une quelconque des revendications 1 à 5 dans un dispositif destiné à protéger la peau, la plaie ou les muqueuses.

## Patentansprüche

1. Film, **dadurch gekennzeichnet, dass** er mindestens ein Polykation und ein polysulfatiertes Oligosaccharid mit einer bis vier Monosaccharid-Einheiten umfasst, wobei der Film eine Dicke zwischen 1 nm und 10 µm aufweist.

2. Film nach Anspruch 1, **dadurch gekennzeichnet, dass** das Polykation in einer Menge von 10-90 Gew.-%, vorzugsweise 30-70 Gew.-%, und das polysulfatierte Oligosaccharid in einer Menge von 10-90 Gew.-%, vorzugsweise 30-70 Gew.-%, bezogen auf das Gewicht des Film, vorliegt.

3. Film nach Anspruch 1 oder 2, **dadurch gekennzeichnet, dass** das Polykation ausgewählt ist aus Poly(L-Lysin), Chitosan, Kollagen, verzweigtem Poly(ethylenimin), Poly(L-Arginin), Poly(allylamin) und Mischungen davon; insbesondere Poly(L-Lysin) und verzweigtem Poly(ethylenimin).

4. Film nach einem der Ansprüche 1 bis 3, **dadurch gekennzeichnet, dass** das polysulfatierte Oligosaccharid aus Saccharose-Octasulfat, vorzugsweise Kalium-Saccharose-Octasulfat, ausgewählt ist.

5. Film nach einem der Ansprüche 1 bis 4, **dadurch gekennzeichnet, dass** er eine Dicke von 10 nm bis 8 µm, weiter bevorzugt 50 nm bis 5 µm, noch weiter bevorzugt 100 nm bis 1 µm aufweist.

6. Film, **dadurch gekennzeichnet, dass** er durch gleichzeitiges oder abwechselndes Zerstäuben mindestens einer Polykationlösung und mindestens einer Lösung eines polysulfatierten Oligosaccharids mit einer bis vier Monosaccharid-Einheiten erhältlich ist, wobei der Film eine Dicke zwischen 1 nm und 10 µm aufweist.

7. Film nach Anspruch 6, **dadurch gekennzeichnet, dass** die zerstäubten Lösungen ein pharmazeutisch akzeptables Medium, vorzugsweise Wasser, enthalten.

8. Film nach Anspruch 6 oder 7, **dadurch gekennzeichnet, dass** die Zerstäubungen gleichzeitig durchgeführt werden.

9. Film, **dadurch gekennzeichnet, dass** er durch abwechselnde Imprägnierung in mindestens einer Polykationlösung und mindestens einer Lösung eines polysulfatierten Oligosaccharids mit einer bis vier Monosaccharid-Einheiten erhältlich ist, wobei der Film eine Dicke zwischen 1 nm und 10 µm aufweist.

10. Film nach Anspruch 9, **dadurch gekennzeichnet, dass** die Lösungen ein pharmazeutisch akzeptables Medium, vorzugsweise Wasser, enthalten.

11. Film zur Verwendung in einem Verfahren zur Freisetzung von polysulfatiertem Oligosaccharid bei Kontakt mit exsudierenden Wunden, wobei das Verfahren insbesondere einen ersten Schritt der Herstellung eines Films gemäß einem der Ansprüche 1 bis 10 und einen zweiten Schritt des Inkontaktbringens des Films mit einer exsudierenden Wunde umfasst.

12. Verwendung des Films nach einem der Ansprüche 1 bis 5 in einer Vorrichtung zum Schutz der Haut, der Wunde oder der Schleimhäute.

## Claims

1. Film **characterised in that** it comprises at least one polycation and one polysulphated oligosaccharide having one to four simple sugar units, said film having a thickness between 1 nm and 10 µm.

2. Film according to claim 1, **characterised in that** the polycation is present in a quantity of 10 to 90% by weight, preferably 30 to 70% and the polysulphated oligosaccharide in a quantity of 10 to 90% by weight, preferably 30 to 70%, with respect to the weight of the film.

3. Film according to claim 1 or 2, **characterised in that** the polycation is selected from poly(L-lysine), chitosan, collagen, branched poly(ethylene imine), poly(L-arginine), poly(allylamine), and mixtures thereof; in particular poly(L-lysine) and branched poly(ethylene imine).

4. Film according to any one of claims 1 to 3, **characterised in that** the polysulphated oligosaccharide is selected from sucrose octasulphates, preferably, potassium sucrose octasulphate.

5. Film according to any one of claims 1 to 4, **characterised in that** it has a thickness of 10 nm to 8 µm, more preferentially of 50 nm to 5 µm, even more preferentially of 100 nm to 1 µm.

6. Film **characterised in that** it is obtainable by simultaneously or alternately spraying at least one polycation solution and at least one polysulphated oligosaccharide solution having one to four simple sugar units, said film having a thickness between 1 nm and 10 µm.

7. Film according to claim 6, **characterised in that** the sprayed solutions comprise a pharmaceutically acceptable medium, preferably water.

8. Film according to claim 6 or 7, **characterised in that** the sprayings are carried out simultaneously.

9. Film **characterised in that** it is obtainable by alternating impregnation in at least one polycation solution and at least one polysulphated oligosaccharide solution having one to four simple sugar units, said film having a thickness between 1 nm and 10 µm.

10. Film according to claim 9, **characterised in that** the solutions comprise a pharmaceutically acceptable medium, preferably water.

11. Film for use thereof in a method for releasing polysulphated oligosaccharides in contact with exudative wounds, said method comprising in particular a first step of preparing a film according to any one of claims 1 to 10, and a second step of placing the film in contact with an exudative wound.

12. Use of the film according to any one of claims 1 to 5 in a device intended to protect the skin, wound, or mucous membranes.
